# EUROPEAN PATENT APPLICATION

(11) **EP 3 819 295 A1**
(43) Date of publication of application: **12.05.2021**
(21) Application number: 19207602.4
(22) Date of filing: 07.11.2019
(51) Int. Cl.: C07D 307/33

(54) **STEREOSELECTIVE SYNTHESIS OF ENANTIOMERICALLY-ENRICHED PANTOLACTONE**

(71) Applicant: DSM IP Assets B.V., 6411 TE Heerlen (NL)
(72) Inventor: BONRATH, Werner, 4303 Kaiseraugst (CH); BOURGEOIS, Frederic, 4056 Basel (CH); MEDLOCK, Jonathan Alan, 4303 Kaiseraugst (CH); SPARR, Christof, 4056 Basel (CH)
(74) Representative: Kurt, Manfred

(57) **Abstract**

The present invention relates stereoselective synthesis of enantiomerically enriched pantolactone of formula (I) or (I').

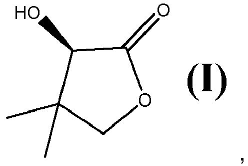

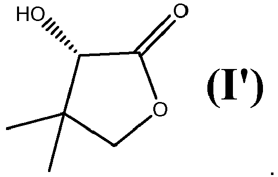

## Description

The present invention relates to a stereoselective synthesis of enantiomerically enriched pantolactone with a specific Iridium catalyst.

Pantolactone has two optically active enantiomers. (R)-pantolactone which is the compound of formula (I) 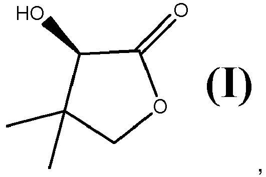 and (S)-pantolactone, which is the compound of formula (I')

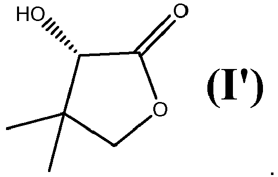

(R)-Pantolactone is a starting material for the synthesis of calcium (R)-pantothenate (compound of formula (II)) 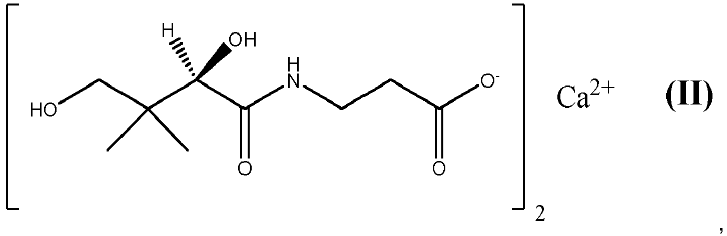 which is the commercial form of pantothenic acid (compound of formula (III))

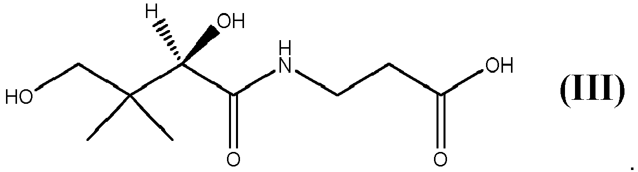

Pantothenic acid, which also known as vitamin B5, is a water-soluble vitamin. Pantothenic acid is an essential nutrient. There are many health benefits of vitamin B5, some of which include a healthy heart, lower stress levels, and applications in skin and hair care.
Instead of pantothenic acid, calcium pantothenate is often used in dietary supplements because, as a salt, it is more stable than pantothenic acid.

Natural sources of vitamin B5 are for example mushrooms, broccoli, cabbage, legumes, salmon, eggs, fish, brewer's yeast, nuts, milk, and dairy products like cheese, wheat, peanuts, soybeans, molasses, and collard greens.

An alternative way to obtain vitamin B5 is by chemical synthesis. An important starting material is, as said above, (R)-pantolactone. A usual way to produce vitamin B5 is the reaction of calcium β-alaninate with (R)-pantolactone in boiling ethanol or methanol.

The other enantiomer of pantolactone, which is (S)-pantolactone, can be used as such or it can be used as intermediate in various synthesis. Alternatively, (S)-pantolactone can also be transformed into (R)-pantolactone.

Due to the importance of vitamin B5 and therefore the relevance of (R)-pantolactone as a starting material to produce vitamin B5 and for the use of (S)-pantolactone, there is always a need for an improved process of production of enantiomerically enriched pantolactone.

Nowadays there are several processes known to produce (R)-pantolactone as well as for (S)-pantolactone. There are chemical as well as biochemical methods to produce (R)-pantolactone as well as (S)-pantolactone. Also, combinations of chemical and biochemical methods are known.
A summary of the most important synthesis of (R)-pantolactone are to be found for example in Ullmans Industrial Chemistry (Vitamins 8 Pantothenic Acid 2012, p.299-308). A common way is to synthesize racemic pantolactone and resolve the enantiomers, however this results in a maximum yield of 50%.

The present invention relates to a two-step stereoselective synthesis of enantiomerically enriched pantolactone, which allows the production of (R)-pantolactone and/or (S)-pantolactone in good yields and good optical purity.
Preferably, the present invention relates to a two-step and one-pot synthesis of (R)-pantolactone in good yields and good optical purity and avoids the need for a resolution step.

The process of production of (R)-pantolactone according to the present invention has the following reaction schemes 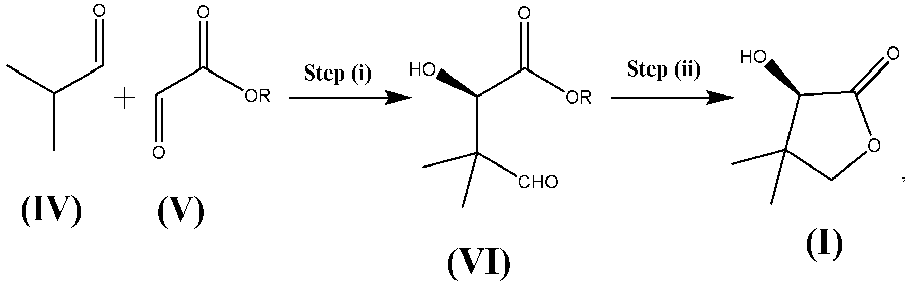 wherein a specific Iridium catalyst is used in step (ii).

The process of production of (S)-pantolactone according to the present invention has the following reaction schemes 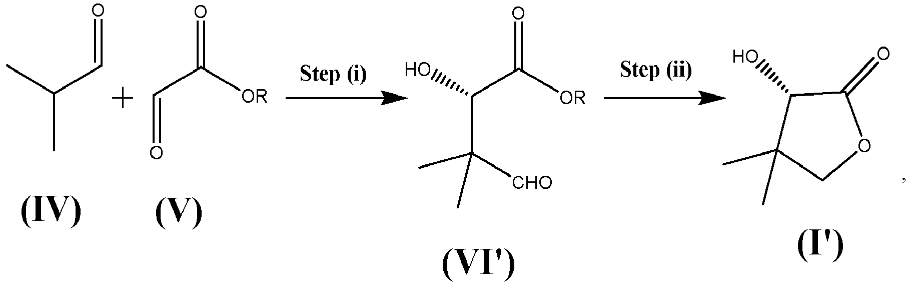 wherein R is a C₁-C₁₀ alkyl moiety, which is substituted or un-substituted alkyl, and wherein a specific Iridium catalyst is used in step (ii).

The first step (step (i)) is carried out in the presence of at least one specific organo-catalyst.

The reaction steps are discussed in more detail below.

### Step (i)

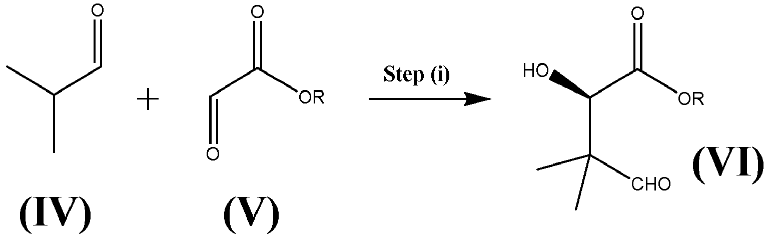

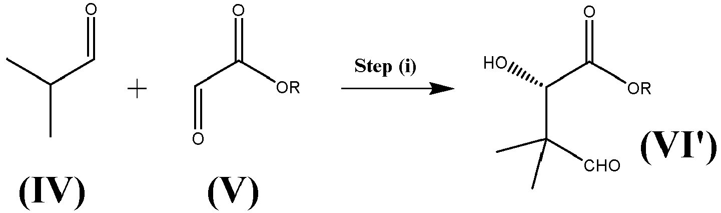

The first step (step (i)) is carried out in the presence of a least one organo-catalyst.

The organo-catalyst has a pyrrolidine ring, which is substituted.

Preferably the organo-catalyst is a compound of formula (VII) 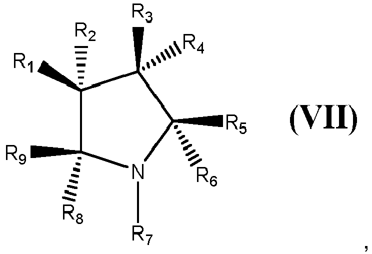 wherein
R₁ is H, CH₃ or OH,
R₂ is H, CH₃ or OH,
R₃ is H or CH₃,
R₄ is H or CH₃,
R₅ and R₆ are independently from each other H or a moiety of formula (VIII) 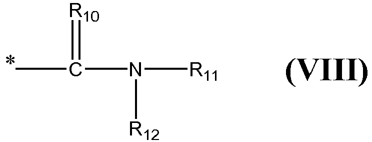 wherein
   R₁₀ is O or NR₁₃, wherein R₁₃ forms together with R₁₁ a tetrazole ring, which can be substituted (by a C₁-C₄ alkyl group)
   R₁₁ is H, CH₃ or forms together with R₁₀ a tetrazole ring, which can be substituted (by a C₁-C₄ alkyl group), and
   R₁₂ is H or CH₃ when R₁₀ and R₁₁ form a tetrazole ring, or
   R₁₂ is an aromatic ring system or a C₂-C₄ alkyl group, which can be substituted, and the * marks the bond to the pyrrolidine ring,
R₇ is H or CH₃,
R₈ is H or CH₃, and
R₉ is H or CH₃,
with the proviso that R₅ is never identical to R₆.

The substituent R₅ or R₆ contains a moiety, that can form a hydrogen bond.

More preferably the organo-catalyst is a compound of formula (VII') 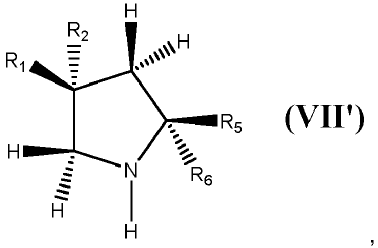 wherein
R₁ is H, CH₃ or OH,
R₂ is H, CH₃ or OH,
R₅ and R₆ are independently from each other H or a moiety of formula (VIII) 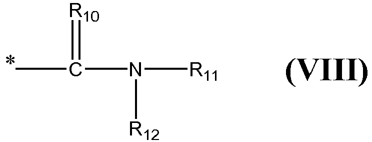 wherein
   R₁₀ is O or NR₁₃, wherein R₁₃ forms together with R₁₁ a tetrazole ring, which can be substituted (by a C₁-C₄ alkyl group)
   R₁₁ is H, CH₃ or forms together with R₁₀ a tetrazole ring, which can be substituted (by a C₁-C₄ alkyl group), and
   R₁₂ is H or CH₃ when R₁₀ and R₁₁ form a tetrazole ring, or
   R₁₂ is an aromatic ring system or a C₂-C₄ alkyl group, which can be substituted, and the * marks the bond to the pyrrolidine ring,
with the provisos that
   R₅ is never identical to R₆, and
   when R₁ is OH or CH₃, then R₂ is H and
   when R₂ is OH or CH₃, then R₁ is H.

An especially preferred organo-catalyst is a compound of formula (VII"a) 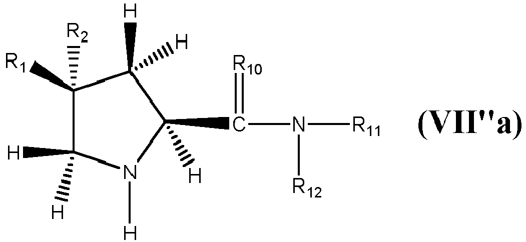 wherein
R₁ is H, CH₃ or OH,
R₂ is H, CH₃ or OH,
R₁₀ is O or NR₁₃, wherein R₁₃ forms together with R₁₁ a tetrazole ring, which can be substituted (by CH₃)
R₁₁ is H, CH₃ or forms together with R₁₀ a tetrazole ring, which can be substituted (by CH₃),
R₁₂ is H or CH₃ when R₁₀ and R₁₁ form a tetrazole ring, or
R₁₂ is an aromatic ring system or a C₂-C₄ alkyl group, which can be substituted
with the provisos that
   when R₁ is OH or CH₃, then R₂ is H and
   when R₂ is OH or CH₃, then R₁ is H.

Another especially preferred organo-catalyst is a compound of formula (VII"b) 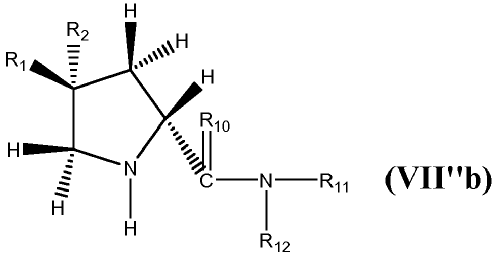 wherein
R₁ is H, CH₃ or OH,
R₂ is H, CH₃ or OH,
R₁₀ is O or NR₁₃, wherein R₁₃ forms together with R₁₁ a tetrazole ring, which can be substituted (by CH₃)
R₁₁ is H, CH₃ or forms together with R₁₀ a tetrazole ring, which can be substituted (by CH₃),
R₁₂ is H or CH₃ when R₁₀ and R₁₁ form a tetrazole ring, or
R₁₂ is an aromatic ring system or a C₂-C₄ alkyl group, which can be substituted
with the provisos that
   when R₁ is OH or CH₃, then R₂ is H and
   when R₂ is OH or CH₃, then R₁ is H.

Especially preferred organo-catalysts of formula (VII) are the following ones of formula (VIIa) - (VIIg) and (ent-VIIa) - (ent-VIIg): 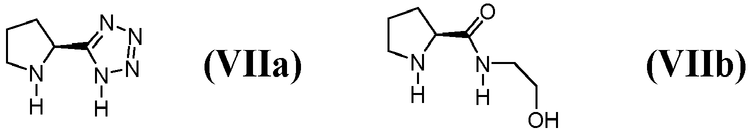 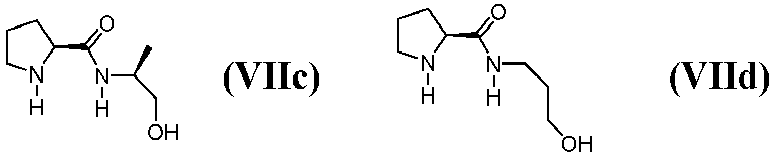 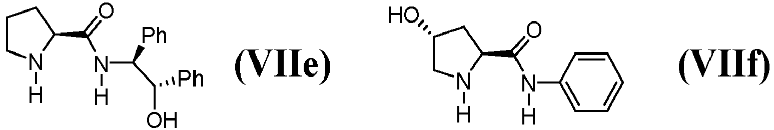 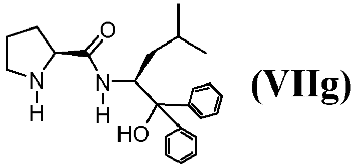 and

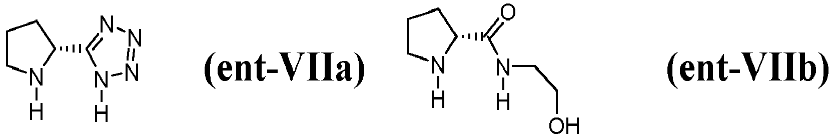

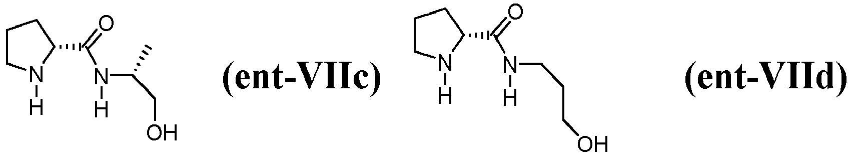

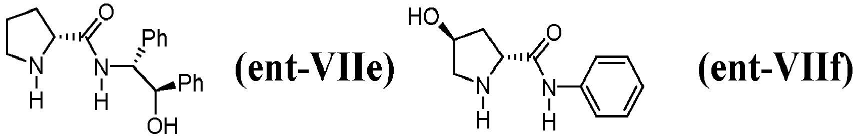

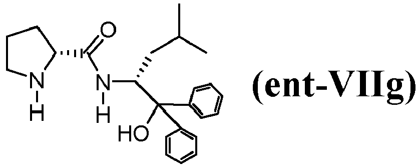

Most preferred are the organo-catalysts of formula (VIIb) and (ent-Vllb) 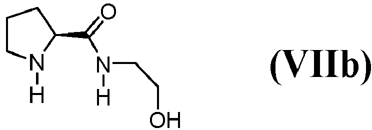 and

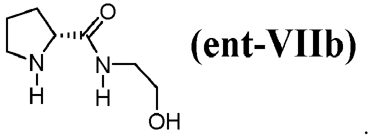

The organo-catalyst as described above are known. They are available commercially or they can be produced according to known methods.

The reaction of step (i) is usually carried out in a solvent (or a mixture of solvents). Suitable solvents are alcohols, hydrocarbons, halogenated hydrocarbons (for example chloroform and dichloromethane), ethers, esters and amides (for example DMF).

Especially preferred are secondary and tertiary alcohols (such as isopropanol (propan-2-ol) and tert- butyl alcohol (2-methylpropan-2-ol)).

The reaction mixture of step (i) should not comprise any water. This means that the water content is kept to a minimum and that no water is added to the reaction mixture of step (i) intentionally.

Therefore, another preferred embodiment of the present invention is a process as described wherein step (i) the reaction mixture does not comprise any water

The reaction is usually carried at temperatures of 0°C - 80°C, preferably 10°C - 40°C, more preferably 20°C - 30°C.

The amount of the organo-catalyst is usually from 0.1 - 10 mol-% (in regard to the starting material). Preferably from 1 - 5 mol-%.

The starting material (the compounds of formula (IV) and (V) are usually added in equimolar amounts. A slight excess of one of the compounds is acceptable as well.

### Step (ii)

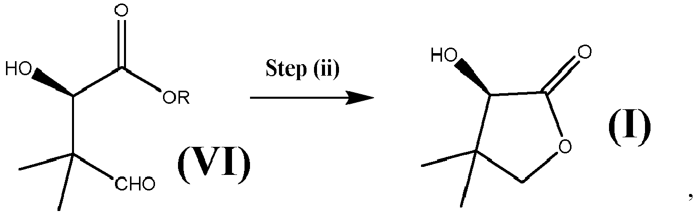

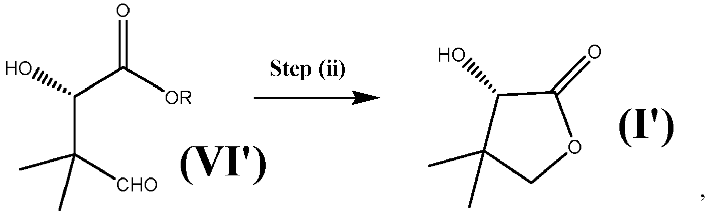

The reaction of step (ii) is a transfer hydrogenation. The reaction of step (ii) is carried out in the presence of a hydrogen donor (such as a formate or an alcohol).
The transfer hydrogenation is catalyzed by a specific Iridium catalyst.

The specific Iridium catalyst can be added as such to the reaction mixture.
Alternatively, the specific Iridium catalyst can be formed by the addition of ligand and by the addition of the Iridium in the form of a salt.
Furthermore, it is also possible that the organo-catalyst of step (i) serves as ligand to form the specific Iridium catalyst used in step (ii). In this case Iridium is added to the reaction mixture in the form of a salt.
These alternative ways how to obtain the specific Iridium catalyst could also be combined (which means that a catalyst can be added as well as a ligand and a transition metal salt).

As stated above, Iridium can be added in form of a salt (such as pentamethylcyclo-pentadienyliridium(III) chloride dimer).

The reaction of step (ii) is usually carried out at elevated temperatures. Preferably, the reaction temperature of step (ii) is between 20°C and 100 °C, more preferably between 30°C and 70 °C.

In the reaction of step (ii), the amount of hydrogen donor is between 1 and 7 mol-eq, preferably between 1 and 5 mol-eq (in regard of the compound of formula (VI) or the compound of formula (VI')).

In the reaction of step (ii), the amount of the iridium salt used to form the catalyst is between 0.02 and 9 mol-%, preferably 0.1 - 9 mol-%, more preferably 0.5 - 4 mol-%, in regard of the compound of formula (VI) or the compound of formula (VI').
When the Iridium catalyst is added as such (not formed in situ), the molar ranges are of the Iridium catalyst is between 0.02 and 9 mol-%, preferably 0.05- 9 mol-%, more preferably 0.5-4 mol-%, in regard of the compound of formula (VI) or the compound of formula (VI').

The following examples serve to illustrate the invention. If not otherwise stated the temperature is given in °C.

### Examples

The organocatalysts used are either commercially available or can be prepared using known methods. One method to prepare a range of organocatalysts is described below.

### General procedure for preparation of various organocatalysts

An oven-dried flask was charged with Cbz-D-proline or Cbz-L-proline (1.00 eq.) or a proline derivative and dry dichloromethane (0.20 mol/L). The solution was cooled to 0 °C and triethylamine (1.00 eq.) and isobutyl chloroformate (1.00 eq.) were added. The mixture was stirred for 0.5 h, and the relevant amine (1.00 eq.) was added. The mixture was warmed to room temperature and stirred until complete conversion (monitored by TLC). The mixture was washed with aq. sat. NH₄Cl, aq. sat. NaHCO₃ and brine. Each aqueous layer was re-extracted with dichloromethane. The combined organic layers were dried over Na₂SO₄, filtered and concentrated in vacuo. The crude intermediate could be purified or used in the following step without further purification. The intermediate (1.00 eq.) was dissolved in MeOH (0.40 mol/L), the flask was flushed with argon three times and Pd/C (10.0 wt.%, 5.00 mol%) was added in one portion. The mixture was evacuated and flushed with hydrogen five times. The black suspension was stirred at room temperature under a hydrogen atmosphere until complete conversion (monitored by TLC). The reaction mixture was filtered over a plug of celite and rinsed with methanol.

### Example 1 - (R)-N-(2-hydroxyethyl)pyrrolidine-2-carboxamide (ent-VIIb)

According to the procedure above: Cbz-D-proline (2.49 g,10.0 mmol, 1.00 eq.), triethylamine (1.41 mL, 10.0 mmol, 1.00 eq.), isobutyl chloroformate (1.30 mL, 10.0 mmol, 1.00 eq.) and ethanolamine (1.21 mL, 10.0 mmol, 1.00 eq.) were reacted to form the intermediate (2.08 g).

The intermediate (2.03 g, 6.94 mmol, 1.00 eq.) and Pd/C (10.0 wt.%, 368 mg, 347 µmol, 5.00 mol%) yielded organocatalyst (ent-Vllb) as a colorless liquid (1.10 g, quant.).

### Example 2: General procedure for step (i) testing various organocatalysts producing (R)-ethyl-2-hydroxy-3,3-dimethyl-4-oxobutanoate (VI)

To a vial containing the organocatalyst (0.01 mmol, 10.0 mol%) 0.20 mL of a stock solution of isobutanal (91.0 µL, 1.00 mmol) and ethyl glyoxalate (50.0 wt.% in toluene, 198 µL, 1.00 mmol) in t-BuOH (2.00 mL) was added. The mixture was stirred at room temperature for 4 - 72 h. Conversion was measured by NMR or GC and enantioselectivity was determined by chiral HPLC.

The results of the experiment are shown in the table below. Where a negative enantioselectivity is reported, this means that the major isomer produced is (S)-isomer. It is clearly understood that if opposite enantiomer of the organocatalyst is used, the identical yield and enantioselectivity for (R)-ethyl-2-hydroxy-3,3-dimethyl-4-oxobutanoate (VI) will be obtained

| Example # | Organocatalyst | Conversion (%) | Enantioselectivity (%) |
|---|---|---|---|
| 2a | VIIa | 88 | -75 |
| 2b | VIIb | 99 | -74 |
| 2c | VIIc | 99 | -70 |
| 2d | VIId | 94 | -64 |
| 2e | VIIe | 54 | -62 |
| 2f | VIIf | 93 | 50 |
| 2g | VIIg | 90 | -58 |

Comparative examples (Comp-A and Comp-B) were performed with organocatalysts A and B under the same conditions.

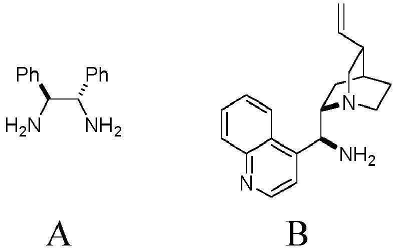

| Example # | Organocatalyst | Conversion (%) | Enantioselectivity (%) |
|---|---|---|---|
| Comp-A | A | 3 | Not determined |
| Comp-B | B | 0 | Not determined |

### Example 3

To a solution of (R)-N-(2-hydroxyethyl)pyrrolidine-2-carboxamide (ent-VIIb, 79.1 mg, 500 µmol, 5.00 mol%) in t-BuOH (10.0 mL), isobutanal (910 µL,10.0 mmol, 1.00 eq.) and ethyl glyoxalate (50.0% in toluene, 1.98 mL,10.0 mmol, 1.00 eq.) were added. The mixture was stirred at room temperature for 24 h. The solvent was removed in vacuo and the residue purified by column chromatography (cyclohexane/ethyl acetate, 4:1) yielding ethyl (R)-2-hydroxy-3,3-dimethyl-4-oxobutanoate (VI) (1.47 g, 84%, 72% ee) as a colorless oil. 1H NMR (400 MHz, CDCl₃) δ = 9.57 (1H, s), 4.32 (1H, s), 4.30-4.18 (2H, m), 3.06 (1H, br), 1.27 (3H, t), 1.14 (3H,s), 1.05 (3H, s). The analytical data was in agreement with an authentic sample.

### General procedure for transfer hydrogenation (step (ii))

The transition metal catalyst or the transition metal salt and the ligand were added to a solution of ethyl (R)-2-hydroxy-3,3-dimethyl-4-oxobutanoate (VI) from example 2. The mixture was degassed, sodium formate was added and the mixture was stirred at the desired temperature until the reduction was complete. The reaction mixture extracted with MTBE and the combined organic phases were dried, filtered and concentrated in vacuo.

### Example 4

To a solution of (R)-N-(2-hydroxyethyl)pyrrolidine-2-carboxamide (ent-VIIb, 7.91 mg, 50.0 µmol, 5.00 mol%) in tBuOH (1.00 mL), isobutanal (72.1 mg, 89 µL, 1.00 mmol, 1.00 eq.) and ethyl glyoxalate (47% in toluene, 211 µL, 1.00 mmol, 1.00 eq.) were added. The mixture was stirred at room temperature for 15 h. Water (5 mL) was added and the solution degassed with argon for 1 h, before (IrCl₂(Cp*))₂ (0.80 mg, 1.00 µmol, 0.1 mol%) and sodium formate (340 mg, 5.00 mmol, 5.00 eq.) were added. The reaction mixture was heated to 40 °C and stirred for 15 h. After addition of aq. HCI (1M, 2 mL) the mixture was extracted with CH₂Cl₂ (3 x 10 mL). The combined organic phases were dried over Na₂SO₄, filtered and concentrated in vacuo. The residue was purified by column chromatography (cyclohexane / ethyl acetate, 2:1) yielding the (R)-pantolactone (80.7 mg, 62%, ratio of enantiomers: 86:14)

### Example 5 -Synthesis of (R) pantolactone (I) with different amounts of Iridium precursor

To a solution of (R)-N-(2-hydroxyethyl)pyrrolidine-2-carboxamide (ent-Vllb, 1 mol/L) at 25°C were added isobutanal (72.1 mg, 89 µL, 1.00 mmol, 1.00 eq.) and ethyl glyoxalate (47% in toluene, 211 µL, 1.00 mmol, 1.00 eq.). The mixture was stirred at 25°C for the indicated time. Water (2:1, water: tBuOH) was added and the solution degassed with argon for 1 h, before (IrCl₂(Cp*))₂ (5.0 - 1.0 µmol, 0.5 - 0.1 mol%) and sodium formate (340 mg, 5.00 mmol, 5.00 eq.) were added. The reaction mixture was heated to the desired temperature and stirred for the stated time. After addition of aq. HCI (1 mol/L, 1 mL) the mixture was extracted with CH₂Cl₂. The combined organic phases were dried over Na₂SO₄, filtered and concentrated in vacuo. The residue was purified by column chromatography.

| **Exp** | **precursor** | **Precursor Loading [mol%]** | **T [°C]** | **T [h]** | **Conv. [%]** |
|---|---|---|---|---|---|
| 1 | (RuCl₂(p-cymene))₂ | 0.50 | rt | 26 | 98 |
| 2 | (RuCl₂(p-cymene))₂ | 0.50 mol% | 40 | 6 | 98 |
| 3 | (RuCl₂(p-cymene))₂ | 0.50 mol% | 60 | 1 | 94 |
| 4 | (RuCl₂(benzene))₂ | 0.50 mol% | 40 | 5 | 92 |
| 5 | (RhCl₂Cp*)₂ | 0.50 mol% | 40 | 2.5 | 97 |
| **6** | **(IrCl₂Cp*)₂** | **0.50 mol%** | **40** | **1** | **99** |
| **7** | **(IrCl₂Cp*)₂** | **0.50 mol%** | **rt** | **4** | **99** |
| **8** | **(IrCl₂Cp*)₂** | **0.25 mol%** | **40** | **2** | **93** |
| **9** | **(IrCl₂Cp*)₂** | **0.10 mol%** | **40** | **5** | **99** |

Examples 6 to 9 are the examples claimed by the present patent claims, whereas claims 1 - 5 are comparison examples.

## Claims

1. Process for the production of a compound of formula (I) 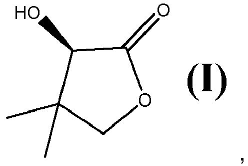 wherein a first step (step (i))
a compound of formula (IV) 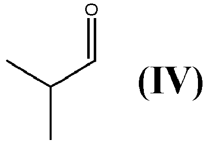 and a compound of formula (V) 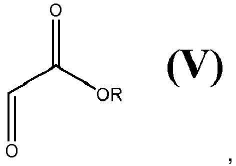 wherein R is a C₁-C₁₀ alkyl moiety, which can be substituted or an un-substituted alkyl, are reacted to form a compound of formula (VI) 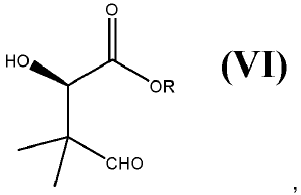 wherein R has the same meaning as defined above,
in the presence of at least one organo-catalyst,
and subsequently in a second step (step (ii))
the compound of formula (I) is formed by a transfer hydrogenation in the presence of hydrogen donor and at least one Iridium catalyst.

2. Process for the production of a compound of formula (I') 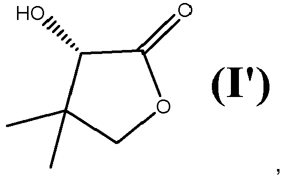 wherein a first step (step (i))
a compound of formula (IV) 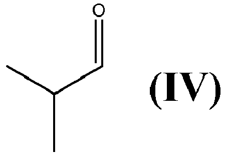 and a compound of formula (V) 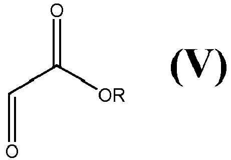 wherein R is a C₁-C₁₀ alkyl moiety, which can be substituted or an un-substituted alkyl
are reacted to form a compound of formula (VI') 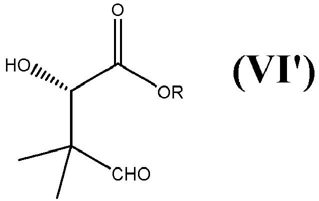 wherein R has the same meaning as defined above,
in the presence of at least one organo-catalyst,
and subsequently in a second step (step (ii))
the compound of formula (I') is formed by a transfer hydrogenation in the presence of hydrogen donor and an iridium catalyst.

3. Process according to claim 1 or claim 2, wherein between 0.02 and 9 mol-%, preferably 0.1 - 9 mol-%, more preferably 0.5 - 4 mol-%, in regard of the compound of formula (VI) or the compound of formula (VI'), of at least one Iridium catalyst is used in step (ii).

4. Process according to claim 1, 2 or claim 3, wherein the organo-catalyst has a pyrrolidine ring, which is substituted.

5. Process according to anyone of the preceding claims, wherein the organo-catalyst is a compound of formula (VII) 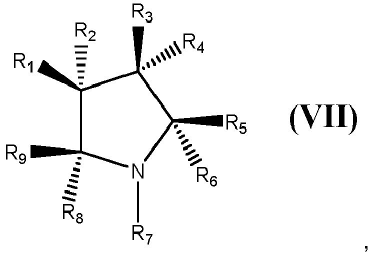 wherein
R₁ is H, CH₃ or OH,
R₂ is H, CH₃ or OH,
R₃ is H or CH₃,
R4 is H or CH₃,
R₅ and R₆ are independently from each other H or a moiety of formula (VIII) 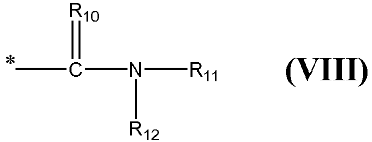 wherein
R₁₀ is O or NR₁₃, wherein R₁₃ forms together with R₁₁ a tetrazole ring, which can be substituted (by a C₁-C₄ alkyl group)
R₁₁ is H, CH₃ or forms together with R₁₀ a tetrazole ring, which can be substituted (a C₁-C₄ alkyl group), and
R₁₂ is H or CH₃ when R₁₀ and R₁₁ form a tetrazole ring, or
R₁₂ is an aromatic ring system or a C₂-C₄ alkyl group, which can be substituted, and the * marks the bond to the pyrrolidine ring,
R₇ is H or CH₃,
R₈ is H or CH₃, and
Rg is H or CH₃,
with the proviso that R₅ is never identical to R₆.

6. Process according to anyone of the preceding claims, wherein the organo-catalyst is a compound of formula (VII') 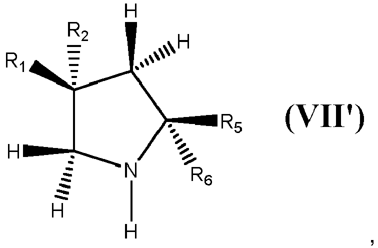 wherein
R₁ is H, CH₃ or OH,
R₂ is H, CH₃ or OH,
R₅ and R₆ are independently from each H or a moiety of formula (VIII) 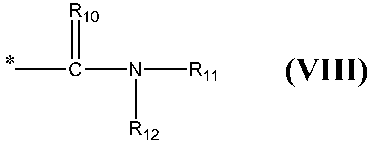 wherein
R₁₀ is O or NR₁₃, wherein R₁₃ forms together with R₁₁ a tetrazole ring, which can be substituted (by a C₁-C₄ alkyl group)
R₁₁ is H, CH₃ or forms together with R₁₀ a tetrazole ring, which can be substituted (by a C₁-C₄ alkyl group), and
R₁₂ is H or CH₃ when R₁₀ and R₁₁ form a tetrazole ring, or
R₁₂ is an aromatic ring system or a C₂-C₄ alkyl group, which can be substituted, and the * marks the bond to the pyrrolidine ring,
with the provisos that
R₅ is never identical to R₆, and
when R₁ is OH or CH₃, then R₂ is H and
when R₂ is OH or CH₃, then R₁ is H.

7. Process according to anyone of the preceding claims, wherein the organo-catalyst is a compound of formula (VII"a) 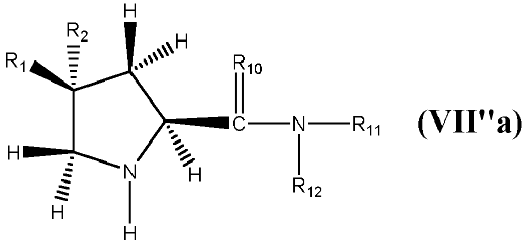 wherein
R₁ is H, CH₃ or OH,
R₂ is H, CH₃ or OH,
R₁₀ is O or NR₁₃, wherein R₁₃ forms together with R₁₁ a tetrazole ring, which can be substituted (by CH₃)
R₁₁ is H, CH₃ or forms together with R₁₀ a tetrazole ring, which can be substituted (by CH₃),
R₁₂ is H or CH₃ when R₁₀ and R₁₁ form a tetrazole ring, or
R₁₂ is an aromatic ring system or a C₂-C₄ alkyl group, which can be substituted with the provisos that
when R₁ is OH or CH₃, then R₂ is H and
when R₂ is OH or CH₃, then R₁ is H.

8. Process according to anyone of the preceding claims, wherein the organo-catalyst is a compound of formula (VII"b) 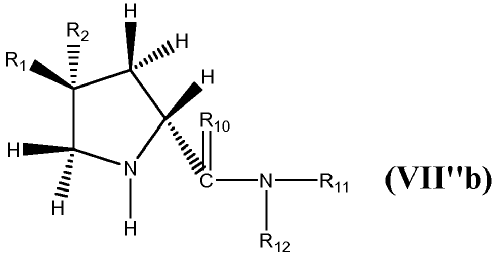 wherein
R₁ is H, CH₃ or OH,
R₂ is H, CH₃ or OH,
R₁₀ is O or NR₁₃, wherein R₁₃ forms together with R₁₁ a tetrazole ring, which can be substituted (by CH₃)
R₁₁ is H, CH₃ or forms together with R₁₀ a tetrazole ring, which can be substituted (by CH₃),
R₁₂ is H or CH₃ when R₁₀ and R₁₁ form a tetrazole ring, or
R₁₂ is an aromatic ring system or a C₂-C₄ alkyl group, which can be substituted with the provisos that
when R₁ is OH or CH₃, then R₂ is H and
when R₂ is OH or CH₃, then R₁ is H.

9. Process according to anyone of the preceding claims, wherein the organo-catalyst is chosen from the group consisting of 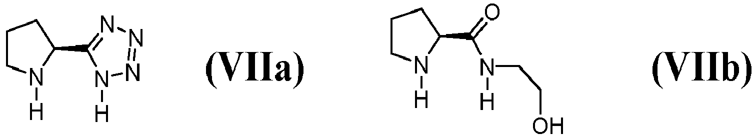 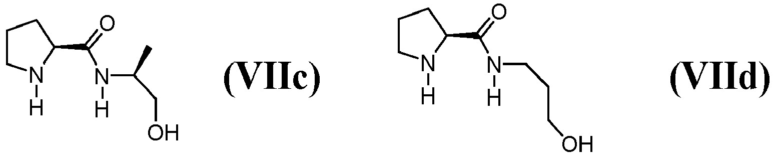 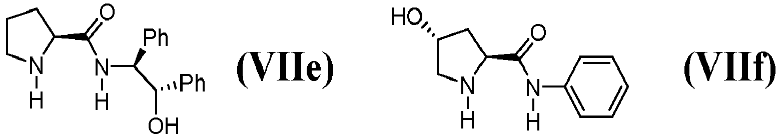 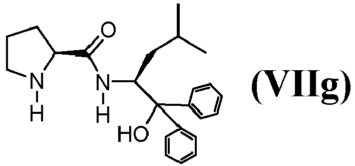 and

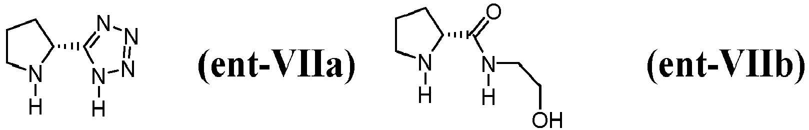

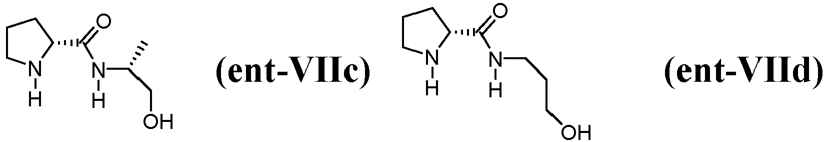

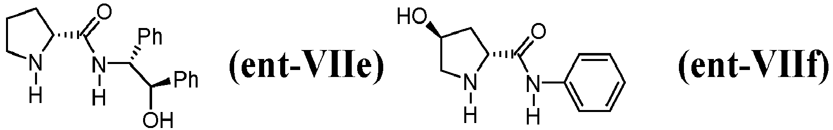

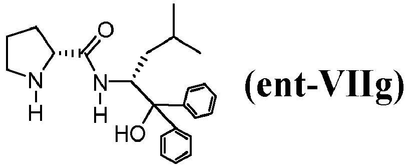

10. Process according to anyone of the preceding claims, wherein the reaction of step (i) is carried out in at least one solvent, preferably alcohols, hydrocarbons, halogenated hydrocarbons (for example chloroform and dichloromethane), ethers, esters and amides (for example DMF).

11. Process according to anyone of the preceding claims, wherein the reaction mixture of step (i) does not comprise any water.

12. Process according to anyone of the preceding claims, wherein the reaction of step (i) is carried at temperatures of 0°C - 80°C, preferably 10°C - 40°C, more preferably 20°C - 30°C.

13. Process according to anyone of the preceding claims, wherein the amount of the organo-catalyst in step (i) is from 0.1 - 10 mol-%, preferably from 1 - 5 mol-%. (regarding the starting material).

14. Process according to anyone of the preceding claims 1 - 13, wherein the transfer hydrogenation of step (ii) is catalyzed by at least one Iridium catalyst, which is formed by the addition of ligand and by the addition of the Iridium in the form of a salt.

15. Process according to anyone of the preceding claims 1 - 14, wherein the transfer hydrogenation of step (ii) is catalyzed by at least one Iridium catalyst, wherein the organo-catalyst of step (i) serves as ligand to form the Iridium catalyst.
